Europäisches Patentamt

European Patent Office

Office européen des brevets

(11) Veröffentlichungsnummer: **0 178 494**
**A1**

(12) # EUROPÄISCHE PATENTANMELDUNG

(21) Anmeldenummer: 85112030.3

(22) Anmeldetag: 23.09.85

(51) Int. Cl.⁴: **C 07 C 37/72**

(30) Priorität: 04.10.84 DE 3436349

(43) Veröffentlichungstag der Anmeldung:
23.04.86 Patentblatt 86/17

(84) Benannte Vertragsstaaten:
AT BE CH DE FR GB IT LI NL

(71) Anmelder: HOECHST AKTIENGESELLSCHAFT
Postfach 80 03 20
D-6230 Frankfurt am Main 80(DE)

(72) Erfinder: Wojtech, Bernhard, Dr.
Kelkheimer Strasse 67
D-6232 Bad Soden am Taunus(DE)

(72) Erfinder: Mayer, Manfred, Dr.
Königsberger Strasse 8
D-6272 Niederhausen/Taunus(DE)

(72) Erfinder: Ott, Karl-Erich
Walddörfer Strasse 263
D-2000 Hamburg 70(DE)

(54) Verfahren zur Extraktion von Phenolen aus wässrigen Lösungen.

(57) Die Erfindung betrifft ein Verfahren zur Extraktion von Phenolen aus wäßrigen Lösungen. Als Extraktionsmittel wird ein Salz aus einem aliphatischen Amin mit einer Gesamtkohlenstoffzahl von mindestens 10 und einer starken Säure verwendet.

EP 0 178 494 A1

**Verfahren zur Extraktion von Phenolen aus wäßrigen Lösungen**

Die Erfindung betrifft ein Verfahren zur Extraktion von Phenolen aus wäßrigen Lösungen.

Derartige Verfahren sind bereits bekannt. Die Phenolextraktion aus Industrieabwässern, die bei der Kohlevergasung, Kohleverflüssigung, Erdölverarbeitung oder Phenolharzherstellung anfallen, wird im großtechnischen Maßstab ausgeführt. Das älteste großtechnische Verfahren, das Benzol-Lauge-Verfahren, verwendet Benzol als Extraktionsmittel und anschließend Natronlauge zur Rückextraktion des Phenols aus der Benzolphase. Infolge des geringen Extraktionsvermögens von Benzol (der Verteilungskoeffizient D = Phenolkonzentration organische Phase:Phenolkonzentration wäßrige Phase beträgt etwa 2,5:1) ist der Extraktionsaufwand (Extraktionsmittelmenge, Stufenzahl) hoch. Eine Verbesserung der Extraktion wird mit dem Phenosolvan-Verfahren (Lurgi) erreicht, das mit Diisopropylether als Extraktionsmittel arbeitet und D-Werte um 30:1 erreicht. Die weitere Entwicklung führte zu noch aktiveren Extraktionsmitteln wie n-Butylacetat (D ≈ 71) und Methylisobutylketon (D ≈ 82). Der Stand der Technik ist in "Ullmanns Encyclopädie der technischen Chemie", 4. Auflage, Band 18, S. 186, beschrieben.

Vorbedingung bei allen Extraktionsverfahren ist die Vorlage eines sauren Abwassers, weil das Phenol nur in seiner undissoziierten sauren Form ($C_6H_5OH$) zu Wasserstoffbrückenbildung fähig ist und so extrahierbar wird.

Neben den oben genannten konventionellen Extraktionsmitteln sind auch höhere primäre, sekundäre und tertiäre Amine, gelöst in aliphatischen oder aromatischen Kohlenwasserstoffen, geeignet (K. Häupke und F. Wolf, in Chem.

Techn. 18 (1966), 405-410). Der Vorteil höherer Amine gegenüber den konventionellen Extraktionsmitteln liegt in ihrer Wasserunlöslichkeit, ihr Nachteil ist die deutlich geringere Extraktionswirkung.

Es wurde nun gefunden, daß sich das Extraktionsvermögen gegenüber Phenolen um das 10 bis 100-fache erhöht, wenn man statt der Amine ihre durch Assoziation mit Mineralsäuren gebildeten Salze (Ionenpaare) als Extraktionsmittel verwendet.

Gegenstand der Erfindung ist ein Verfahren zur Extraktion von Phenolen aus wäßrigen Lösungen, dadurch gekennzeichnet, daß man als Extraktionsmittel ein Salz aus einem aliphatischen Amin mit einer Gesamtkohlenstoffzahl von mindestens 10 und einer starken Säure verwendet. Vorzugsweise wird als starke Säure eine Mineralsäure eingesetzt.

Die Aminsalzbildung erfolgt einfach und quantitativ durch Vermischen einer starken Säure mit dem Amin, wobei bei Mineralsäuren diese aus der wäßrigen Phase unter Ionenpaarbildung in die organische Phase übergeht. Das Gleichgewicht dieser "Neutralisation" liegt ganz auf der Seite des Aminsalzes. Die Gleichgewichtskonstanten betragen $10^4$ bis $10^8$, je nach dem Amin, der Säure und evtl. dem Verdünnungsmittel. Solche Aminsalze haben die Zusammensetzung $(RH_2NH)X$, $(R_2HNH)X$, $(R_3NH)X$, wobei X das Anion der Säure ist, wie z.B. $Cl^-$, $SO_4^=$, $NO_3^-$, $ClO_4^-$ und $HPO_4^=$. Die aliphatischen Amine können primär, sekundär oder tertiär sein, und der oder die Alkylreste haben insgesamt mindestens 10, vorzugsweise 20 bis 50 C-Atome und können geradkettig, cyclisch oder verzweigt sein. Besonders bevorzugt unter den genannten Aminen sind wegen ihrer geringen chemischen Reaktivität die tertiären Amine, wie Tri-n-octylamin, Tri-isooctylamin, Tri-n-decyclamin, Tri-isodecylamin, Tri-n-dodecyclamin, Tri-isododecylamin bzw. deren Gemische.

0178494

Die Aminsalze können unverdünnt vorgelegt werden, sie können aber zur Viskositätsverminderung auch mit einem Lösemittel verdünnt sein. Auch eine Teilumwandlung - mindestens 10 % - des Amins in das Aminsalz ist möglich, wobei das restliche (freie) Amin als Verdünnungsmittel wirkt.

Die Säure zur Aminsalzbildung kann auch der wäßrigen phenolhaltigen Phase zugegeben werden, die dann im Kontakt mit dem Amin das extraktionswirksame Aminsalz bildet.

Die Phenolkonzentration in der wäßrigen Phase kann bis zur Sättigungskonzentration reichen. Neben dem unsubstituierten Phenol ($C_6H_5OH$) können auch Alkylphenole oder mehrwertige Phenole (z.B. Resorcin) und Polyphenole (z.B. Hydroxydiphenyle, Dihydroxydiphenyle oder Dihydroxydiphenylmethane) erfindungsgemäß extrahiert werden. Besonders wichtig ist die Extraktion des unsubstituierten Phenols, ferner die der Kresole und Xylenole, sowie der weiteren Alkylphenole, deren Alkylrest oder Alkylreste zusammen bis zu 5 C-Atome haben.

Die wäßrigen Lösungen der Phenole können auch andere gelöste organische oder anorganische Verbindungen und suspendierte Teilchen enthalten, sofern diese die Extraktion nicht stören. Eine vorhergehende extraktive Abtrennung störender organischer Verunreinigungen ist manchmal erforderlich. Das erfindungsgemäße Extraktionsverfahren erlaubt auch die Gewinnung von Phenolen bei großer Verdünnung, insbesondere aus Abwässern, z.B. aus solchen mit einem Phenolgehalt um 1 Gew.-% oder darunter.

Die Extraktion kann beispielsweise kontinuierlich im Gegenstrom in einem mehrstufigen Extraktor erfolgen, wobei infolge der hohen Verteilungskoeffizienten der Extraktionsmittelbedarf, bezogen auf die wäßrige Phase, gering ist, sodaß das Verhältnis wäßrige Phase:organische Phase (Phasenvolumenverhältnis) bis etwa 50:1 betragen kann.

Auf diese Weise ist eine starke Anreicherung der Phenole im Extrakt möglich. Das große Extraktionsvermögen des Extraktionsmittels erlaubt es aber auch, einstufig im Rührkessel zu extrahieren, wobei das Verhältnis wäßrige Phase:organische Phase bis etwa 10:1 betragen kann.

Die Isolierung der Phenole aus dem Extrakt auf destillativem Weg ist bei den flüchtigen Phenolen, wie $C_6H_5OH$, möglich. Dazu ist es vorteilhaft, das Aminsalz ohne flüchtiges Verdünnungsmittel einzusetzen. Günstig ist es auch, das Aminsalz eines hochsiedenden Amins, z.B. Tri-n-octylamin (Kp ≈ 350°C), verdünnt mit dem freien Amin zu verwenden, d.h. das Amin vorher nur partiell in das Aminsalz zu überführen. Die Rückextraktion der Phenole aus dem Extrakt durch Alkalilaugebehandlung unter Phenolatbildung ist ebenfalls geeignet.

Die Erfindung soll anhand folgender Beispiele erläutert werden. Dabei ist OP = organische Phase und WP = wäßrige Phase.

Beispiel 1

Ein phenolhaltiges Abwasser aus der Phenolharzherstellung, enthaltend 0,56 mmol/g Phenole, wurde im Gegenstrom in einem Mischabsetzer bei Raumtemperatur mit einer Lösung von 1 mmol Aminsulfat pro Gramm (hergestellt aus Tri-n-octylamin und Tri-n-octylamin 50:50) in einem Gemisch aus $C_{10}$-$C_{11}$-Alkylbenzolen (unter dem Namen Solvesso[R] 150 im Handel) bei einem Phasenvolumenverhältnis wäßrige Phase : organische Phase von 1:1 extrahiert. Die Phenolkonzentration in der Wasserphase sank bereits bei einstufiger Extraktion auf einen Wert von 0,03 mmol/g, entsprechend einer Extraktionsausbeute von 95 %. Reines Amin statt Aminsulfat bei gleicher Konzentration in Solvesso 150 und unter sonst gleichen Bedingungen ergab erst nach 10 Stufen eine Phenolkonzentration in der Wasserphase von 0,046 mmol/g, entsprechend einer Extraktionsausbeute von 92 %. Das Phenol aus dem Aminsulfat-Solvesso-Extrakt wurde im Dünnschichtverdampfer bei 90°C im Vakuum (0,1 mbar) abgetrennt.

Beispiel 2

Proben einer wäßrigen Lösung von 5 Gew.-% (0,531 mmol/g) Phenol wurden in Schüttelgefäßen bei Raumtemperatur und verschiedenen Phasenvolumenverhältnissen mit einem Extraktionsmittel ins Gleichgewicht gesetzt, das einmal erfindungsgemäß aus einer Lösung von Aminsalzen in Solvesso 150 bestand, und dann zum Vergleich mit der freien Aminkomponente in Solvesso 150 bei gleicher molarer Aminkonzentration. Als Aminkomponente wurde ein Gemisch aus Tri-n-octylamin und Tri-n-decylamin (50:50) eingesetzt. Folgende Aminsalze wurden geprüft: Aminsulfat $(R_3NH)_2SO_4$, Aminchlorid $(R_3NH)Cl$ und Aminhydrogenphosphat $(R_3NH)_2HPO_4$. Die Herstellung dieser Salze erfolgte jeweils durch Mischung des im Solvesso 150 gelösten Amins mit einer äquivalenten Menge Säure in wäßriger Lösung in Schüttelgefäßen unter analytischer Kontrolle. Die Ergebnisse sind Tabelle 1 zu entnehmen.

<u>Tabelle 1</u>

| Nr. | Phasenvolumen-verhältnis WP : OP | Extraktionsmittel in Solvesso 150 | (mmol/g) | Phenolkonzentration (mmol/g) OP | WP | Verteilungs-koeffizient |
|---|---|---|---|---|---|---|
| 1 | 2:1 | Amin | 0,5 | 0,824 | 0,155 | 5,3 |
| 2 | 2:1 | Aminhydrogenphosphat | 0,5 | 0,984 | 0,057 | 17,3 |
| 3 | 2:1 | Aminchlorid | 0,5 | 0,972 | 0,060 | 16,2 |
| 4 | 2:1 | Aminsulfat | 0,5 | 1,028 | 0,037 | 27,8 |
| 5 | 1:1 | Amin | 0,5 | 0,489 | 0,094 | 5,2 |
| 6 | 1:1 | Aminhydrogenphosphat | 0,5 | 0,550 | 0,012 | 46 |
| 7 | 1:1 | Aminchlorid | 0,5 | 0,563 | 0,010 | 56 |
| 8 | 1:1 | Aminsulfat | 0,5 | 0,570 | 0,0037 | 154 |
| 9 | 1:2 | Amin | 0,5 | 0,272 | 0,052 | 5,2 |
| 10 | 1:2 | Aminhydrogenphosphat | 0,5 | 0,291 | 0,0031 | 94 |
| 11 | 1:2 | Aminchlorid | 0,5 | 0,293 | 0,0021 | 140 |
| 12 | 1:2 | Aminsulfat | 0,5 | 0,294 | 0,00059 | 498 |
| 13 | 2:1 | Amin | 1,0 | 0,907 | 0,112 | 8,1 |
| 14 | 2:1 | Aminsulfat | 1,0 | 1,103 | 0,0047 | 235 |
| 15 | 1:1 | Amin | 1,0 | 0,522 | 0,065 | 8,0 |
| 16 | 1:1 | Aminsulfat | 1,0 | 0,577 | 0,0014 | 412 |
| 17 | 1:2 | Amin | 1,0 | 0,284 | 0,035 | 8,1 |
| 18 | 1:2 | Aminsulfat | 1,0 | 0,294 | 0,00059 | 498 |

0178494

Beispiel 3

In einstufigen Verteilungsversuchen mit einem Phenolabwasser aus der Phenolharzherstellung wurde das Extraktionsvermögen von reiner Aminkomponente (Tri-n-octylamin und Tri-n-decylamin 50:50) verglichen mit dem der gleichen Aminkomponente, die zu 70 mol.-% in Aminsulfate überführt worden war, unter sonst gleichen Bedingungen bei Raumtemperatur. Das Abwasser hatte eine Konzentration an Phenolen von 0,296 mmol/g. Die Ergebnisse zeigt Tabelle 2.

Tabelle 2

| Nr. | Phasenvolumen-verhältnis WP : OP | Extraktionsmittel A = reine Amine B = 70 mol.% Amin-sulfate 30 mol.% Amine | Phenolkonzentration (mmol/g) OP | WP | Verteilungs-koeffizient |
|---|---|---|---|---|---|
| 1 | 1:1 | A | 0,300 | 0,0423 | 7,1 |
| 2 | 1:1 | B | 0,323 | 0,0072 | 44,9 |
| 3 | 2:1 | A | 0,565 | 0,0579 | 9,8 |
| 4 | 2:1 | B | 0,639 | 0,0088 | 72,6 |
| 5 | 4:1 | A | 0,917 | 0,1013 | 9,1 |
| 6 | 4:1 | B | 1,234 | 0,0129 | 95,5 |

Die wesentlich höheren Verteilungskoeffizienten zeigen das überlegene Extraktionsvermögen des Aminsulfats. So wurde z.B. bei dem Experiment Nr. 4 mit Aminsulfat einstufig bereits eine Extraktionsausbeute von 97 % erreicht, während unter den gleichen Bedingungen mit dem freien Amin (Experiment Nr. 3) nur 80 % zu erzielen waren.

Auch eine 6-stufige Gegenstromextraktion bei gleichem Phasenvolumenverhältnis bewirkte beim freien Amin erst eine Extraktionsausbeute von 93,6 % bei einer Konzentration von 0,02 mmol Phenol/g in der Wasserphase. Dafür war aber ein Extraktor mit 6 theoretischen Stufen erforderlich, während für die Extraktion mit Aminsulfat nur ein Rührkessel benötigt wurde.

Beispiel 4

Es wurden 100 ml einer wäßrigen Lösung hergestellt, die 5 Gew.-% Phenol und 71 mmol $H_2SO_4$ (= 6,963 g) enthielt. Das ist diejenige Menge Schwefelsäure, die benötigt wird, um 100 ml Amin (Tri-n-octylamin-/Tri-n-decylamin 50:50) zu 70 mol.% in das Aminsulfat zu überführen. Die wäßrige schwefelsaure Phenollösung und 100 ml des Amins wurden dann bei Raumtemperatur ins Gleichgewicht gesetzt und der Phenolgehalt der beiden Phasen analysiert. Gefunden wurde in der organischen Phase 0,526 mmol Phenol/g, in der wäßrigen Phase 0,00105 mmol Phenol/g, das entspricht einem Verteilungskoeffizienten von 501/1. Man sieht aus dem extremen Verteilungskoeffizienten, daß es zur Aminsulfatbildung gekommen ist. Die zur Aminsulfatbildung erforderliche Säure kann also, wie dieses Beispiel zeigt, auch der wäßrigen Phase zugesetzt werden.

Beispiel 5

Vorgelegt waren als wäßrige Phasen: eine wäßrige Phenollösung von 0,531 mmol/g (5,0 Gew.-%), eine wäßrige gesättigte p-Kresollösung von 0,215 mmol/g (2,3 Gew.-%) und eine wäßrige Resorcinlösung von 0,443 mmol/g (4,9 Gew.-%). Verglichen wurde das Extraktionsvermögen von reiner Aminkomponente (Tri-n-octylamin-/Tri-n-decylamin 50:50) mit dem der gleichen Aminkomponente die zu 70 mol.% in Aminsulfat umgewandelt war. Tabelle 3 zeigt die Ergebnisse.

Tabelle 3

| Nr. | Phasenvolumenverhältnis WP : OP | Extraktionsmittel A = reine Amine B = 70 mol.% Aminsulfate, 30 mol.% Amine | Extraktstoff Name | Konz. in mmol/g OP | WP | Verteilungskoeffizient |
|---|---|---|---|---|---|---|
| 1 | 1:1 | A | Phenol | 0,569 | 0,512 | 11,1 |
| 2 | 1:1 | B | Phenol | 0,621 | 0,0015 | 414 |
| 3 | 2:1 | A | Phenol | 1,019 | 0,0889 | 11,5 |
| 4 | 2:1 | B | Phenol | 1,179 | 0,0034 | 347 |
| 5 | 4:1 | A | Phenol | 1,676 | 0,1418 | 11,8 |
| 6 | 4:1 | B | Phenol | 1,774 | 0,0177 | 100 |
| 7 | 1:1 | A | Resorcin | 0,175 | 0,306 | 0,57 |
| 8 | 1:1 | B | Resorcin | 0,492 | 0,0029 | 171 |
| 9 | 1:1 | A | Kresol | 0,226 | 0,012 | 19 |
| 10 | 1:1 | B | Kresol | 0,240 | 0,00064 | 375 |

**0178494**

## PATENTANSPRÜCHE:

1. Verfahren zur Extraktion von Phenolen aus wäßrigen Lösungen, dadurch gekennzeichnet, daß man als Extraktionsmittel ein Salz aus einem aliphatischen Amin mit einer Gesamtkohlenstoffzahl von mindestens 10 und einer starken Säure verwendet.

2. Verfahren nach Anspruch 1, dadurch gekennzeichnet, daß man ein Salz eines tertiären Amins mit einer Gesamtkohlenstoffzahl von 20 bis 50 einsetzt.

3. Verfahren nach Anspruch 1 oder 2, dadurch gekennzeichnet, daß man ein Aminsalz der Salzsäure, Salpetersäure, Schwefelsäure oder Phosphorsäure einsetzt.

4. Verfahren nach einem der Ansprüche 1 bis 3, dadurch gekennzeichnet, daß das Aminsalz verdünnt in einem Lösemittel eingesetzt wird.

Europäisches
Patentamt

**EUROPÄISCHER RECHERCHENBERICHT**

**0178494**
Nummer der Anmeldung

EP 85 11 2030

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A- 406 150 (RÜTGERSWERKE AG)<br>* Ansprüche 1-3; Seite 1, Zeilen 11-28 * | 1-4 | C 07 C 37/72 |
| | --- | | |
| A | DE-A-1 030 351 (THE DISTILLERS CO.)<br>* Anspruch 1 * | 1 | |
| | ----- | | |

**RECHERCHIERTE SACHGEBIETE (Int. Cl.4)**

C 07 C 37/00

Der vorliegende Recherchenbericht wurde für alle Patentansprüche erstellt.

| Recherchenort<br>DEN HAAG | Abschlußdatum der Recherche<br>10-01-1986 | Prüfer<br>KLAG M.J. |
|---|---|---|